# EUROPÄISCHE PATENTANMELDUNG

(11) **EP 1 754 493 A1**
(43) Veröffentlichungstag der Anmeldung: **21.02.2007**
(21) Anmeldenummer: 06013543.1
(22) Anmeldetag: 30.06.2006
(51) Int. Cl.: A61L 9/16, A61L 9/12

(54) **Verfahren zum Entfernen von übelriechenden Dämpfen und Gasen**

(30) Priorität: 17.08.2005 DE 102005038767
(71) Anmelder: Biothys GmbH, 77731 Willstätt (DE)
(72) Erfinder: Wuest, Robert, 67190 Rosheim (FR); Moeckers, Jean Marie, 67190 Mutzig (FR)
(74) Vertreter: Wagner, Jutta

(57) **Zusammenfassung**

Die Erfindung betrifft ein Verfahren zum Entfernen von übelriechenden Dämpfen und Gasen beim Abfüllen von Kohlenwasserstoff-Flüssigkeiten, die aus einem Tank in den Einfüllstutzen eines Transportmittels gepumpt wird. Dies geschieht dadurch, dass die aus dem Einfüllstutzen heraustretenden übelriechenden Dämpfe und Gase seitlich weggeblasen und eventuell neutralisiert werden.

## Beschreibung

Die Erfindung betrifft ein Verfahren zum Entfernen von übelriechenden Dämpfen und Gasen beim Abfüllen von Flüssigkeiten, die aus einem Tank in den Einfüllstutzen eines Transportmittels für die Flüssigkeit gepumpt werden, oder die aus dem Transportmittel in einen Behälter entleert werden.

Wenn in Raffinerien oder in Zwischenlager-Unternehmen Kohlenwasserstoff-Flüssigkeiten, z.B. Schweröl oder Benzin aus einem großen Vorratstank in Tanklastzüge umgefüllt werden, wird das Einfüllrohr in den Einfüllstutzen des Lastzugs im allgemeinen von Hand eingeführt. Dabei kommt es bei dem mit dem Einfüllen und der Überwachung beauftragten Personal zu erheblicher Geruchsbelästigung und mitunter zu Gesundheitsgefährdung, da aus dem Einfüllstutzen aufgrund des ansteigenden Flüssigkeitsspiegels übelriechende Dämpfe und Gase austreten. Eine Absaugung dieser Dämpfe ist oft nicht möglich, da diese - insbesondere bei Schweröl und Bitumen - zu klebrigen Tröpfchen kondensieren, die die Absauganlage verstopfen würden.

Ein ähnliches Problem tritt auf, wenn beim Entleeren des Transportmittels an dessen Unterseite die Flüssigkeit ausläuft und der Einfüllstutzen zum Druckausgleich geöffnet wird. Auch dabei können übelriechende Dämpfe und Gase aus dem Einfüllstutzen nach oben austreten.

Der Erfindung lag daher die Aufgabe zugrunde, ein einfaches und wirksames Verfahren zur Beseitigung dieses Missstandes bereitzustellen.

Diese Aufgabe wird erfindungsgemäß dadurch gelöst, dass die aus dem Einfüllstutzen austretenden übelriechenden Dämpfe und Gase seitlich weggeblasen und dadurch verdünnt werden.

Das Verfahren ist vor allem für Ölraffinerien und Zwischenlager geeignet, wo große Mengen Leichtöl oder Benzin, aber auch flüssige Düngemittel und Chemikalien, z.B. Styrol oder Ammoniak, von Vorratstanks in Lastzüge umgefüllt werden. Das Verfahren ist insbesondere auch für das Abfüllen von Bitumen, Asphalt und Schweröl geeignet, die durch Erhitzen, vorzugsweise auf 60 bis 120°C , verflüssigt wurden.

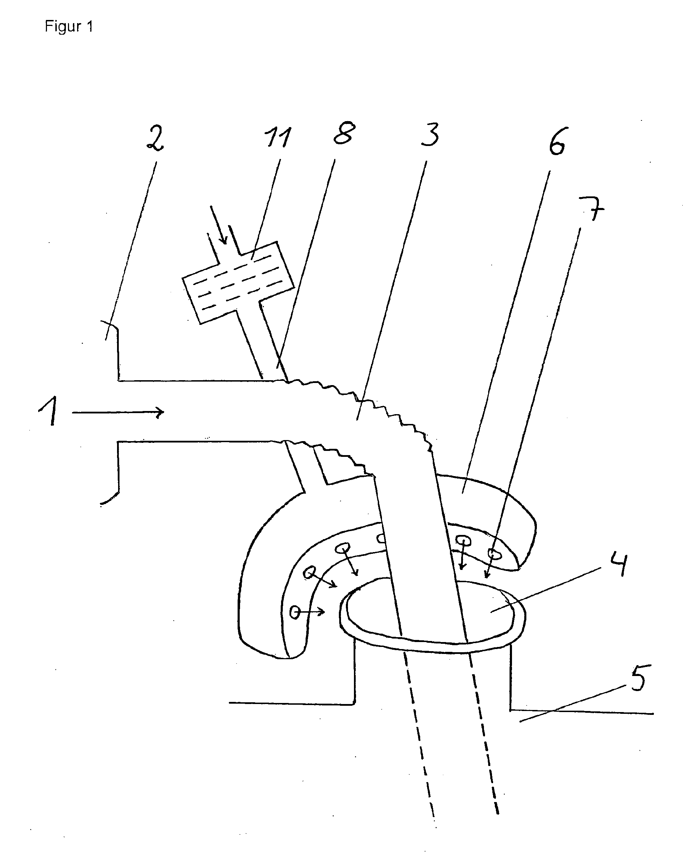

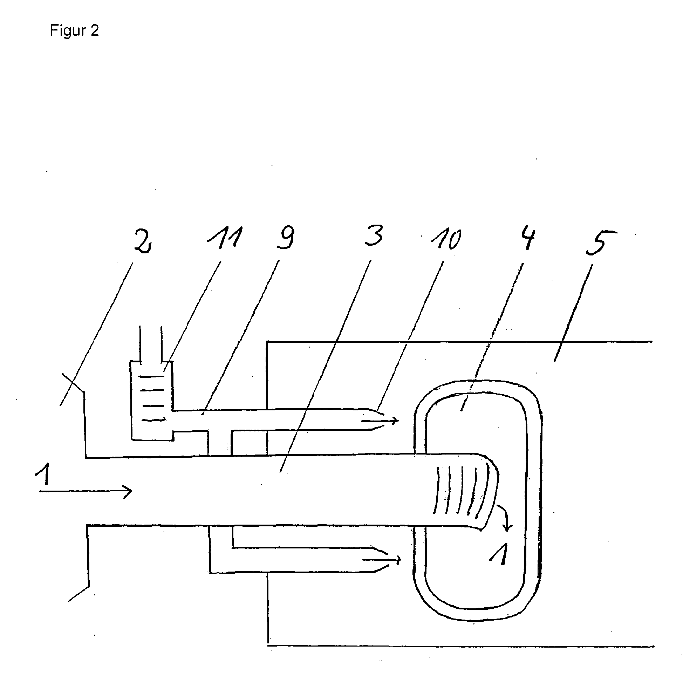

Die Erfindung soll anhand der in Figuren 1 und 2 dargestellten bevorzugten Ausführungsformen näher erläutert werden.

In beiden Figuren ist mit **(1)** die Flüssigkeit, die aus dem Tank **(2)** über ein vorzugsweise bewegliches Einfüllrohr **(3)** in den Einfüllstutzen **(4)** des Transportmittels **(5)** gepumpt wird, bezeichnet.

Bei der Ausführungsform nach Figur 1 wird an die Öffnung des Einfüllstutzens **(4)** eine gebogene Blasvorrichtung **(6)** herangeführt, deren Krümmung in etwa der Krümmung des Einfüllstutzens entspricht. Die gebogene Blasvorrichtung kann bananenförmig oder viertel- bis halbringförmig sein. Die Blasvorrichtung weist an der dem Einfüllstutzen zugewandten Seite mehrere Öffnungen **(7)** auf, aus denen die Luft bzw. der Wasserdampf ausgeblasen wird. Die Öffnungen sind an der Blasvorrichtung konzentrisch so angebracht, dass die aus ihnen austretende Luft bzw. der Wasserdampf die aus dem Einfüllstutzen heraustretenden übelriechenden Dämpfe und Gase unmittelbar seitlich wegblasen kann. Die Luft bzw. der Wasserdampf wird mittels eines Gebläses über ein Rohr **(8)** in die Blasvorrichtung eingeführt.

Bei der Ausführungsform nach Figur 2 (Ansicht von oben) besteht die Blas-vorrichtung aus einem oder mehreren, vorzugsweise zwei bis vier Rohren **(9)** mit Öffnungen **(10),** aus denen die Luft bzw. der Wasserdampf ausgeblasen wird. Die Rohre **(9)** sind unter dem Einfüllrohr **(3)** oder um dieses herum so angeordnet, dass die aus ihnen austretende Luft bzw. der Wasserdampf die aus dem Einfüllstutzen **(4)** heraustretenden übelriechenden Dämpfe und Gase unmittelbar seitlich wegblasen kann.

In beiden Fällen sollte der Abstand der Öffnungen **(7)** bzw. **(10)** vom Rand des Einfüllstutzens **(4)** möglichst gering sein und vorzugsweise 5 bis 100 cm, insbesondere 10 bis 80 cm betragen. Die Blasvorrichtungen sollten zweckmäßigerweise - von der Seite gesehen - etwas oberhalb des Einfüllstutzens, vorzugsweise 2 bis 20 cm oberhalb, angeordnet sein, so dass die austretenden übelriechenden Dämpfe und Gase weggeblasen werden können.

Bei der Variante der Erfindung, bei der die Flüssigkeit aus dem Transportmittel nach unten in einen Behälter abläuft, fällt natürlich das Einfüllrohr **(3)** weg. Ansonsten kommen auch hier beide Ausführungsformen in Frage.

Selbstverständlich sind auch andersartige Ausführungsformen der Erfindung möglich.

Bei einer bevorzugten weiteren Ausgestaltung der Erfindung werden die übelriechenden Dämpfe und Gase nicht nur seitlich weggeblasen, sondern gleichzeitig neutralisiert. Dazu werden die Rohre **(8)** bzw. **(9)** mit einem Kasten **(11)** verbunden, in dem sich flüchtige, an einem Trägermaterial adsorbierte aktive Agenzien befinden. Das Trägermaterial ist zweckmäßigerweise auf Netzen oder Gittern abgelegt. Die aktiven Agenzien werden durch die Luft bzw. den Wasserdampf, die durch den Kasten durchgeblasen werden, desorbiert und freigesetzt. Sie können mit den weggeblasenen übelriechenden Dämpfen und Gasen reagieren oder diese maskieren und dadurch weitgehend neutralisieren. Dieses Prinzip ist in der Publikation WO 03/063920 ausführlich beschrieben. Grundsätzlich können die aktiven Agenzien in flüssiger Form auch ohne Trägermaterial auf Unterlagen verteilt eingesetzt werden.

## Patentansprüche

1. Verfahren zum Entfernen von übelriechenden Dämpfen und Gasen beim Einfüllen von Flüssigkeiten, die aus einem Tank über ein Einfüllrohr in den Einfüllstutzen eines Transportmittels für die Flüssigkeit gepumpt wird, oder beim Entleeren der Flüssigkeit aus dem Transportmittel in einen Behälter, **dadurch gekennzeichnet, dass** an die Öffnung des Einfüllstutzens eine Vorrichtung herangeführt wird, aus der Luft und/oder Wasserdampf in Querrichtung zur Öffnung des Einfüllstutzens über diese hinweg geblasen wird, wodurch die aus dem Einfüllstutzen nach oben austretenden übelriechenden Dämpfe und Gase seitlich abgeführt und verdünnt werden.

2. Verfahren nach Anspruch 1, **dadurch gekennzeichnet, dass** die Flüssigkeit eine Kohlenwasserstoff- Flüssigkeit, wie Leichtöl oder Benzin ist.

3. Verfahren nach Anspruch 1, **dadurch gekennzeichnet, dass** die Flüssigkeit Schweröl, Asphalt oder Bitumen ist, die erhitzt und **dadurch** verflüssigt wurden.

4. Verfahren nach Anspruch 1, **dadurch gekennzeichnet, dass** die Flüssigkeit flüssige Düngemittel oder Chemikalien sind.

5. Verfahren nach einem der Ansprüche 1 bis 4, **dadurch gekennzeichnet, dass** die Vorrichtung eine gebogene Blasvorrichtung ist mit einer Krümmung, die etwa der Krümmung des Einfüllstutzens entspricht, und dass die Blasvorrichtung über ihre Breite an der dem Einfüllstutzen zugewandten Seite eine oder mehrere Öffnungen aufweist, aus denen Luft und/oder Wasserdampf ausgeblasen wird.

6. Verfahren nach Anspruch 5, **dadurch gekennzeichnet, dass** die Luft bzw. der Wasserdampf über ein Rohr in die gebogene Blasvorrichtung hinein eingeführt wird.

7. Verfahren nach einem der Ansprüche 1 bis 4, **dadurch gekennzeichnet, dass** die Vorrichtung eine Blasvorrichtung ist, welche aus einem oder mehreren Rohren mit Öffnungen besteht, aus denen Luft und/ oder Wasserdampf ausgeblasen wird.

8. Verfahren nach Anspruch 5, 6 oder 7, **dadurch gekennzeichnet, dass** der Abstand der Öffnungen der Blasvorrichtung vom Rand des Einfüllstutzens 5 bis 100 cm beträgt.

9. Verfahren nach einem der Ansprüche 6 bis 8, **dadurch gekennzeichnet, dass** die Rohre mit einem Kasten verbunden sind, in dem sich flüchtige, vorzugsweise an ein Trägermaterial adsorbierte, aktive Agenzien befinden, die durch die durchgeblasene Luft bzw. den Wasserdampf freigesetzt werden und mit den übelriechenden Dämpfen und Gasen reagieren oder diese maskieren.
